(19) Europäisches Patentamt

European Patent Office

Office européen des brevets

(11) **EP 1 721 871 A1**

(12) **EUROPEAN PATENT APPLICATION**
published in accordance with Art. 158(3) EPC

(43) Date of publication:
**15.11.2006 Bulletin 2006/46**

(51) Int Cl.:
*C02F 11/04* [(2006.01)]   *C02F 3/00* [(2006.01)]
*C02F 3/28* [(2006.01)]   *C12P 3/00* [(2006.01)]
*C12P 5/02* [(2006.01)]   *B09B 3/00* [(2006.01)]
*C10L 3/00* [(2006.01)]

(21) Application number: **05719128.0**

(22) Date of filing: **15.02.2005**

(86) International application number:
**PCT/JP2005/002226**

(87) International publication number:
**WO 2005/077845 (25.08.2005 Gazette 2005/34)**

(84) Designated Contracting States:
**AT BE BG CH CY CZ DE DK EE ES FI FR GB GR
HU IE IS IT LI LT LU MC NL PL PT RO SE SI SK TR**

(30) Priority: **16.02.2004 JP 2000038882**

(71) Applicant: **Sapporo Breweries Limited
Tokyo 150-8522 (JP)**

(72) Inventors:
• **MITANI, Yutaka**
c/o Sapporo Breweries Limited
Yaizu-shi,
Shizuoka 4250013 (JP)
• **NISHIO, Naomichi**
hiroshima-shi,
Hiroshima 7398530 (JP)

(74) Representative: **Vossius & Partner
Siebertstrasse 4
81675 München (DE)**

(54) **PROCESS FOR PRODUCING BIOGAS**

(57) A production method of a biogas of the present invention comprises carrying out hydrogen fermentation of a subject solution containing organic matter with the use of a hydrogen fermentation microorganism, in which according to a correlation between a concentration of a predetermined substrate in a liquid to be processed containing an organic matter and a rate of consumption of the substrate by a hydrogen-fermenting microorganism a maximum tolerable concentration of the substrate consumable by the hydrogen-fermenting microorganism is determined in advance and in which in the actual hydrogen formation step, the concentration of the substrate in the liquid is maintained at one not higher than the maximum tolerable concentration. According to the production method of a biogas of the present invention, hydrogen fermentation can be performed sufficiently smoothly without any treatment of the material involving consumption of thermal energy such as heating/warming.

**Fig.1**

## Description

## Technical Field

The present invention relates to a production method of biogas which is useful as an energy gas.

## Background Art

Anaerobic fermentation using microorganisms has been known as a method of converting biomasses such as organic wastes and organic waste water into energy. The anaerobic fermentation is a fermentation scheme in which an acid generating step from an organic matter and a methane generating step of generating methane from an organic acid generated by the acid generating fermentation usually proceed as multiple parallel fermentation, whereby a fermentation gas mainly composed of methane can be obtained as an energy gas.

However, the energy obtained by boiler combustion of methane is heat, so that it is not suitable for applications requiring no heat utilization, but is limited to those directly utilizing the heat of combustion, those converting the heat into steam, and the like. Methane fuel batteries convert the resulting energy into electric power, whereby their usage is broader than the heat utilization. However, a so-called reforming reaction for generating hydrogen from methane requires a reformer and heating of a material methane gas. Usually, the heat of combustion of methane is utilized as a heat source therefor, and its thermal energy is collected by a technique such as warm water manufacture from the viewpoint of effective energy utilization. As a result, the methane fuel battery utilization also needs to use thermal energy.

In the acid generating step in anaerobic fermentation, on the other hand, a fermentation gas mainly composed of hydrogen has been known to occur. Hydrogen is quite useful, since it is not problematic in terms of thermal energy like methane. For example, hydrogen is advantageous in that no reforming reaction is necessary when used in a fuel battery, so that a large part of generated hydrogen can be fed to the fuel battery and converted into electric power. Hence, a technique for generating a fermentation gas mainly composed of hydrogen and a fermentation gas mainly composed of methane separately from each other at the time of anaerobic fermentation has been proposed (see, for example, Patent Documents 1, 2, and 3).

[Patent Document 1] Japanese Patent Application Laid-Open No. SHO 61-8200
[Patent Document 2] Japanese Patent Application Laid-Open No. 2001-149983
[Patent Document 3] Japanese Patent Application Laid-Open No. 2003-135089

## Disclosure of the Invention

## Problem to be Solved by the Invention

However, even the above-mentioned conventional methods are not easy to perform hydrogen fermentation smoothly at a practical level. Namely, it has been reported that there are cases where biomasses to become materials contain contaminant bacteria such as lactic acid bacteria other than hydrogen-generating bacteria, and these contaminant bacteria inhibit the hydrogen fermentation (Noike et al., Inhibition of hydrogen fermentation of organic wastes by lactic acid bacteria, International Journal of Hydrogen Energy, Vol. 27, pp. 1367-1371, 2002).

As a method overcoming this problem, the above-mentioned Patent Document 3 discloses a method of inactivating hydrogen fermentation inhibiting bacteria in a material by subjecting a biomass to be hydrogen-fermented to a heating/warming process beforehand. However, thermal energy is necessary for such a heating/warming process, whereby it does not become a fundamental solution. Patent Documents 1 and 2 do not mention the above-mentioned problem at all. Namely, the first object of the fermenting process for collecting an energy gas from a biomass employed as a material is to process wastes or waste water of the biomass. Therefore, the process must decompose the biomass so as to reduce its volume greatly and lower the load due to the waste water. In this operation, because of characteristics of waste processing and waste water processing, an excess of energy input for the operation and process greatly lowers the processing efficiency and remarkably deteriorates the industrial usefulness.

In view of such circumstances, it is an object of the present invention to provide a production method of a biogas, which can sufficiently smoothly perform hydrogen fermentation or hydrogen fermentation and methane fermentation when using a hydrogen-fermenting microorganism to carry out the hydrogen fermentation from an organic matter such as a biomass as a material or when carrying out the methane fermentation after the hydrogen fermentation, without subjecting the material to a treatment of the material involving consumption of thermal energy such as heating/warming.

**Means for Solving the Problem**

The inventors conducted diligent studies in order to achieve the above-mentioned object and, as a result, have initially found that whether the hydrogen generation by a hydrogen-fermenting microorganism and growth of the hydrogen-fermenting microorganism or the growth of a microorganism group such as lactic acid bacteria which adversely affects the hydrogen fermentation and fermentation by the microorganism group become dominant depend on the concentration of a predetermined substrate contained in a liquid to be processed. Further studies based on this finding have revealed that the above-mentioned problem is overcome when the concentration of the substrate in the liquid to be processed is kept within an appropriate range in practice according to a correlation between the concentration of the substrate and the rate of consumption of the substrate by the hydrogen-fermenting microorganism, whereby the present invention is achieved.

Namely, the present invention provides a production method of a biogas, the method comprising a first step of determining, according to a correlation between a concentration of a predetermined substrate in a liquid to be processed containing an organic matter and a rate of consumption of the substrate by a hydrogen-fermenting microorganism, a maximum tolerable concentration of the substrate consumable by the hydrogen-fermenting microorganism; and a second step of generating a biogas mainly composed of hydrogen by causing the hydrogen-fermenting microorganism to hydrogen-ferment the liquid to be processed while keeping the substrate in the liquid to be processed at a concentration not higher than the maximum tolerable concentration.

In the case where the maximum tolerable concentration of a substrate consumable by a hydrogen-fermenting microorganism is determined beforehand according to the correlation between the concentration of the substrate in a liquid to be processed containing an organic matter and the rate of consumption of the substrate by the hydrogen-fermenting microorganism, and the concentration of the substrate in the liquid is kept at a level not higher than the maximum tolerable concentration when performing hydrogen fermentation in practice as such, the organic matter, which is a material, is predominantly consumed by the hydrogen-fermenting microorganism, whereby the growth of microorganisms (contaminant microorganisms) such as lactic bacteria adversely affecting the growth or activity of the hydrogen-fermenting microorganism and their resulting fermentation are sufficiently suppressed. Therefore, the present invention can sufficiently prevent the contaminant microorganisms from inhibiting the hydrogen fermentation without a treatment of the material involving consumption of thermal energy such as heating/warming, whereby the hydrogen fermentation can be performed sufficiently smoothly.

Preferably, in the present invention, the substrate to become an index of the hydrogen fermentation is a glucide. Using a glucide as an index, determining its maximum tolerable concentration, and keeping the glucide concentration at a level not higher than the maximum tolerable concentration when performing the hydrogen fermentation in practice as such can more reliably prevent contaminant microorganisms from inhibiting the hydrogen fermentation, whereby the hydrogen fermentation can be carried out more smoothly.

Preferably, the production method of a biogas in accordance with the present invention further comprises a third step of generating a fermentation gas mainly composed of methane by causing a methane-fermenting microorganism to methane-ferment the fermented liquid after the hydrogen fermentation in the second step. When the fermented liquid after the hydrogen fermentation in the second step is subjected to methane fermentation, contaminant products are sufficiently restrained from inhibiting the methane fermentation. Therefore, a fermentation gas mainly composed of hydrogen and a fermentation gas mainly composed of methane can be generated separately and sufficiently smoothly. Also, providing the third step is quite useful in terms of reducing the volume of organic wastes, lowering the environmental load due to organic waste water, etc.

The present invention provides a production method of a biogas, the method comprising the step of generating a biogas mainly composed of hydrogen by performing hydrogen fermentation while adding a hop or hop component to a liquid to be processed containing an organic matter so as to inactivate a contaminant microorganism inhibiting hydrogen generation without affecting a growth or activity of a hydrogen-fermenting microorganism.

Hops and hop components have been known to exhibit antibacterial actions against wide ranges of microorganisms. For example, Simpson, W.J. et al. reported antibacterial activities against lactic acid bacteria, Lactobacillus brevis (Simpson, W.J. et al., Factors affecting antibacterial activity of hops and their derivatives, J. Appl. Bacteriol., vol. 72, pp. 327-334, 1992), whereas Plollach G et al. reported that hop beta acid restrained microorganisms from generating lactic acid, nitrous acid, acetic acid, and butyric acid (Plollach G et al., Einsatz von Hophenprodukten als Bacteriosticum in der Zuckerindustrie, Zuckerindustrie, vol. 121, pp. 919-926, 1996; Hein, W. et al., Neue Erkenntnisse beim Einsatz von Hopfenprodukten in der Zuckerindustrie, Zuckerindustrie, vol. 122, pp. 940-949, 1997; Plollach, G et al., Neue Erkenntnisse zur Losungmikrobieller Probleme in Zuckerfabriken, Zuckerindustrie, vol. 124, pp. 622-637, 1999). On the other hand, cases with resistivity were also reported, whereby effective functions have not always been established conventionally. For example, Simpson, W.J. et al. reported that genera Pediococcus and Lactobacillus exhibited hop resistivities (Simpson, W.J. et al., Cambridge Prize Lecture, Studies on the Sensitivity of Lactic Acid Bacteria to Hop Bitter Acids, J. Inst. Brew., vol. 99, pp. 405-411, 1993); Sami, M. reported that Lactobacillus brevis strain exhibited a hop resistivity (Sami, M., Lactic Acid Bacteria Deteriorating Beer, Journal of the Brewing Society of Japan, vol. 94, pp. 2-9, 1999); and so forth. The inventors studied this point and, as a result, have verified that appropriately setting conditions such as method of utilization and amount of use of a hop or hop component effectively suppresses the activity of microorganisms which adversely affect the activity of hydrogen-fermenting microorganisms and does not inhibit the growth and activity of the hydrogen-fermenting microorganisms, whereby the possibility of effectively utilizing the hop or hop component in hydrogen fermentation has been clarified. The above-mentioned production method of a biogas sufficiently prevents contaminant microorganisms from inhibiting hydrogen fermentation without performing a treatment of the material involving consumption of thermal energy such as heating/warming, thereby making it possible to carry out hydrogen fermentation sufficiently smoothly.

**Effect of the Invention**

As mentioned above, according to the present invention, when using a hydrogen-fermenting microorganism to carry out the hydrogen fermentation from an organic matter as a material, the hydrogen fermentation can be performed sufficiently smoothly without a treatment of the material involving consumption of thermal energy such as heating/ warming.

**Brief Description of the Drawings**

Fig. 1 is a block diagram showing an example of biogas generating apparatus favorably used in the present invention.

Fig. 2 is a graph showing the correlation between the number of days of fermentation and the hydrogen and carbon dioxide concentrations in the fermentation gas, which was obtained by Example 1.
Fig. 3 is a graph showing the correlation between the number of days of fermentation and the hydrogen and carbon dioxide concentrations in the fermentation gas, which was obtained by Example 3.
Fig. 4 is a graph showing the correlation between the number of days of fermentation and the hydrogen and carbon dioxide concentrations in the fermentation gas, which was obtained by Example 4.

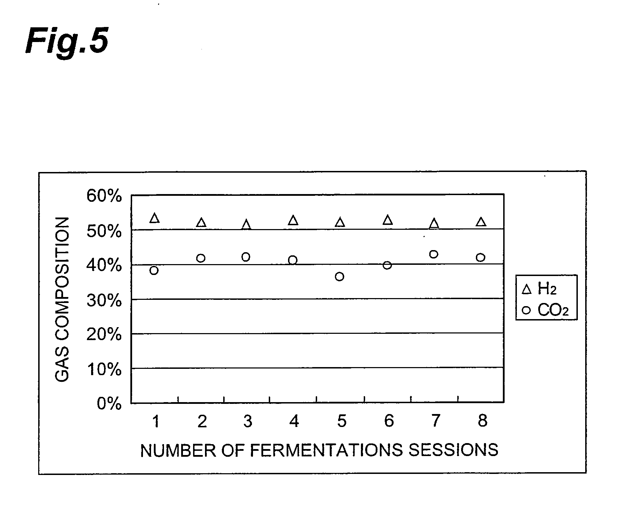

Fig. 5 is a graph showing the correlation between the number of fermentation sessions and the hydrogen and carbon dioxide concentrations in the fermentation gas, which was obtained by Example 6.
Fig. 6 is a graph showing the correlation between the number of days of fermentation and the hydrogen and carbon dioxide concentrations in the fermentation gas, which was obtained by Example 8.
Fig. 7 is a graph showing the correlation between the species of material supply liquids and the hydrogen and carbon dioxide concentrations in the fermentation gas, which was obtained by Example 9.
Fig. 8 is a graph showing the correlation between the number of days of fermentation and the methane and carbon dioxide concentrations in the fermentation gas, which was obtained by Example 10.

**Explanations of letters or numerals**

1···a hydrogen fermentation tank, 2···a methane fermentation tank, L1 to L5 ··· lines

**Best Modes for Carrying Out the Invention**

In the following, preferred embodiments of the present invention will be explained in detail.

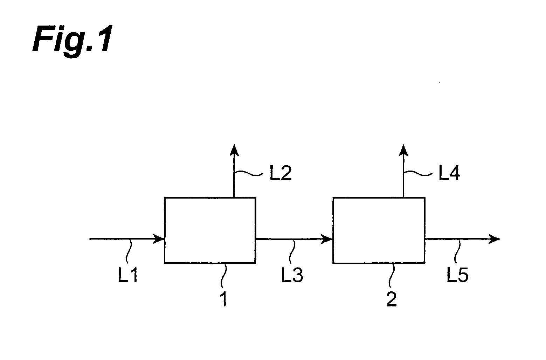

Fig. 1 is a block diagram showing an example of biogas production apparatus preferably used in the present invention. The apparatus shown in Fig. 1 comprises a hydrogen fermentation tank 1 and a methane fermentation tank 2, thereby carrying out hydrogen/methane two-stage fermentation by a continuous operation.
The hydrogen fermentation tank 1 is provided with a line L1, whereas a liquid to be processed containing an organic matter is fed to the hydrogen fermentation tank 1 by way of the line L1. The liquid to be processed is not limited in particular as long as it contains an organic matter which can be hydrogen-fermented by a hydrogen-fermenting microorganism. The hydrogen fermentation tank 1 is useful for processing biomasses such as organic wastes and organic waste water in order to acquire energy gasses from reusable organic resources among others, and is preferably employed for processing beer brewery waste water, bakery wastes, etc. in particular.
A hydrogen-fermenting microorganism is contained in the hydrogen fermentation tank 1. The hydrogen-fermenting microorganism performs hydrogen fermentation from the organic matter in the liquid to be processed. Examples of the hydrogen-fermenting microorganism include anaerobic microorganisms such as Clostridia, Methylotrophs, Methanogens, Rumen Bacteria, and Archaebacteria; facultative anaerobic microorganisms such as Escherichia coli and Enterobacter; aerobic microorganisms such as Alcaligenes and Bacillus; photosynthetic bacteria; and Cyanobacteria.

The hydrogen-fermenting microorganism may be either an isolated microorganism or a mixed microorganism group (microflora) suitable for hydrogen production. For example, the hydrogen fermentation by an anaerobic microorganism group can be performed by supplying an organic material such as a biomass to a fermentation tank containing a hydrogen-fermenting microorganism under a condition with a pH of about 6.0 to 7.5 and a temperature of about 20° to 70°C. When hydrogen fermentation is effected by such a hydrogen-fermenting microorganism, a fermentation gas (biogas) mainly composed of hydrogen ($H_2$) and carbon dioxide ($CO_2$) occurs, while organic acids such as acetic acid, butyric acid, and lactic acid are generated. For example, glucose is decomposed by an action of a hydrogen-fermenting microorganism into acetic acid ($CH_3COOH$), hydrogen, and carbon dioxide according to the following expression (1):

$$C_6H_{12}O_6 + 2H_2O \rightarrow 2CH_3COOH + 2CO_2 + 4H_2 \qquad (1)$$

When causing the hydrogen-fermenting microorganism to perform hydrogen fermentation in the present invention, according to a correlation between the concentration of a predetermined substrate in the liquid to be processed and the rate of consumption of the substrate by the hydrogen-fermenting microorganism, a maximum tolerable concentration of the substrate consumable by the hydrogen-fermenting microorganism is initially determined. Here, the substrate to become an index is not restricted in particular as long as it correlates with the hydrogen generation by the hydrogen-fermenting microorganism and the growth of the hydrogen-fermenting microorganism. A preferred substrate is a glucide.
The "maximum tolerable concentration of the substrate" refers to the maximum value of concentration of the substrate allowing the substrate to be consumed predominantly by the hydrogen-fermenting microorganism for the hydrogen fermentation. Namely, when the concentration of the substrate in the liquid to be processed in the hydrogen fermentation tank 1 is kept at a level not higher than the maximum tolerable concentration, the substrate is predominantly consumed by the hydrogen-fermenting microorganism, whereby the hydrogen fermentation can be performed sufficiently smoothly. When the concentration of the substrate exceeds the maximum tolerable concentration, lactic acid bacteria and the like existing in the organic matter such as a biomass remarkably inhibit hydrogen fermentation activities, thereby suppressing the hydrogen generation or the growth of hydrogen-fermenting microorganism.
The maximum tolerable concentration of the substrate can be determined by the following procedure, for example. First, a plurality of liquids to be processed containing respective concentrations of a substrate different from each other are prepared, hydrogen fermentation is performed by using them, and amounts of hydrogen generation at that time are determined. The concentration of the substrate can be adjusted by changing the dilution ratio of the liquid to be processed, or adding the substrate to the liquid to be processed. When the substrate to become an index is a glucide, for example, the glucide concentration in the liquid to be processed can be enhanced if a polymer polysaccharide such as cellulose, hemicellulose, or starch; an oligosaccharide such as maltotriose, cellobiose, or cellotriose; a monosaccharide such as pentose or hexose; or the like is added thereto.
Next, measured amounts of hydrogen generation are plotted against concentrations of the substrate, whereby a correlation curve of the substrate concentration vs. hydrogen generation amount is obtained. In this correlation curve, the hydrogen generation amount usually tends to increase as the substrate concentration increases, and decrease after attaining a maximum value at a certain concentration. Since the hydrogen generation amount depends on the rate of consumption of the substrate by the hydrogen-fermenting microorganism, the concentration yielding the maximum value of hydrogen generation amount in the correlation curve becomes the maximum tolerable concentration of the substrate.
Adding a hop or hop component to the liquid to be processed here can effectively suppress activities of a microorganism group such as lactic acid bacteria which adversely affect the hydrogen fermentation. Antibacterial actions due to the hop or hop component do not affect activities of the hydrogen-fermenting microorganism. Therefore, the addition of the hop or hop component to the liquid to be processed can enhance the maximum tolerable concentration of the substrate, thereby further improving the efficiency at the time of performing the hydrogen fermentation in practice. While the liquid to be processed after the hydrogen fermentation (fermented liquid) is subjected to methane fermentation which will be explained later, the methane fermentation can be performed more smoothly if this fermented liquid contains a hop or hop component.
Preferably employed as the hop or hop component are chemically modified hops such as hop strobiles, hop pellets, hop extracts, isomerized hop pellets, and tetrahydroisohumulones; hop $\alpha$-acid; hop $\beta$-acid; and the like.
According to thus determined maximum tolerable concentration, the hydrogen fermentation is performed in practice. Namely, the substrate concentration in the supplied liquid to be processed, respective rates at which the liquid to be processed flows in and out, etc. are adjusted such that the concentration of the substrate in the hydrogen fermentation tank 1 is not higher than the maximum tolerable concentration, and a hop or hop component is further added thereto if necessary, whereby the hydrogen fermentation is performed by the hydrogen-fermenting microorganism. When the organic matter as a material has the same quality, and fermentation conditions such as temperature and pH within the hydrogen fermentation tank are unchanged, the amount of growing microorganism existing in the hydrogen fermentation tank is substantially held constant. In continuous operations, the liquid to be processed is continuously fed to the hydrogen fermentation tank while being continuously discharged therefrom, whereby it is desirable that the liquid to be processed be continuously supplied while taking account of the flow-in and flow-out of the liquid to be processed, the consumption of the organic matter (or substrate) by microorganisms, etc. Using microorganism immobilization can make the microorganism keeping amount substantially constant without being influenced by fluctuations in the material concentration (i.e., substrate concentration) of the liquid to be processed within the hydrogen fermentation tank (fermented liquid) or fluctuations in the rate at which the liquid to be processed flows in or out.

The material balance within the hydrogen fermentation tank 1 in a continuous operation can be represented by the following expression (2):

$$V(dS/dt) = FS_o - FS - V(-dS/dt)_C \qquad (2)$$

In expression (2), V is the volume of the liquid to be processed in the hydrogen fermentation tank, So is the substrate concentration in the liquid to be processed flowing in, and dS/dt is the amount of fluctuation in substrate concentration per unit time. Subscript C indicates that $(-dS/dt)_C$ is the amount of fluctuation due to the consumption by microorganisms. F is the rate at which the liquid to be processed is supplied and the rate at which the fermented liquid flows when a fixed volume operation is assumed. Namely, the left side of expression (2) is the amount of fluctuation in substrate consumption per unit time per fermentation tank, the first term on the right side is the amount of the substrate flowing in, the second term on the right side is the amount of the substrate flowing out, and the third term on the right side is the amount of consumption of the substrate by microorganisms.

In a fermentation operation of generating an energy gas from a biomass, it is important to maximize the energy gas generation rate per fermentation tank. In this regard, from the viewpoint of fermentation rate, it is desirable that the microorganism keeping amount in the fermentation tank be made as large as possible, and that the fermentation tank volume be utilized as much as possible. When the fermentation operation is regulated at a predetermined temperature and a predetermined pH, the rate at which the substrate is consumed by microorganisms depends on the microorganism keeping amount in the fermentation tank assuming that there are no disturbing elements such as mingling of toxic matters and lack of essential nutrients, whereby the third term on the right side, i.e., $V(-dS/dt)_C$, is kept at a value as large as possible in practice. Examples of techniques for holding the microorganism keeping amount in the fermentation tank as much as possible include a process of immobilizing microorganisms in a microorganism carrier; and a process of forming flocculating microorganism masses, and filling the fermentation tank with them or floating them therein. Though there is a technique in which microorganisms are grown and kept at a high concentration in a floating state without immobilizing them, the microorganism concentration is susceptible to the flow-in of the material liquid and the rate at which the fermented liquid flows out, whereby it is desirable to employ a microorganism immobilizing technique.

In a fermenting operation of producing an energy gas from a biomass or the like, it is important in terms of apparatus efficiency that that the biomass, which is a fermentation material, to be processed stably for a long period, so as to produce a fermentation gas stably. Further, from the viewpoint of waste water processing and the like, the load concentration in the effluent must be kept from fluctuating. Therefore, it is not desirable for variable terms on the left side of expression (2) to fluctuate unstably, so that zero-fluctuation operations are important.

Here, keeping the biomass material concentration such that the biomass material is not used for the growth and fermentation of the microorganism group such as lactic acid bacteria is synonymous with keeping the substrate concentration S in the effluent at a level not higher than the maximum tolerable concentration.

When the fluctuation on the left side is set to zero according to the view mentioned above, expression (2) can be rewritten as expression (3a) or (3b):

$$(S - S_o)/(-dS/dt)_c = V/F \qquad (3a)$$

$$F = V \times (-dS/dt)_c / (S - S_o) \qquad (3b)$$

Assuming that V and $(-dS/dt)_c$ are constant since they should be as large as possible and held constant as mentioned above, it will be sufficient if the material liquid supply and the fermented liquid flow-out rate F are calculated from the right side of expression (3b) with respect to the substrate concentration So in the flow-in material liquid in order to keep the substrate concentration S in the effluent at a predetermined level and operate the substrate consumption variable term per fermentation tank (left side of expression (2)) so as to prevent it from fluctuating.

Performing the hydrogen fermentation by the hydrogen-fermenting microorganism as such generates a fermentation gas (biogas) mainly composed of hydrogen and carbon dioxide, and produces an organic acid such as acetic acid, butyric acid, or lactic acid. Thus generated biogas is taken out of the hydrogen fermentation tank 1 by way of a line L2. Though the biogas can be used in a fuel battery or the like while still in a mixed gas of hydrogen and carbon dioxide, a film separator equipped with a palladium film which passes hydrogen therethrough and blocks carbon dioxide may be used so as to isolate and collect hydrogen with a high purity from the mixed gas. Highly pure hydrogen can also be obtained by causing the mixed gas to pass through an alkali solution and making the alkali solution absorb carbon dioxide. On the other hand, the processed liquid (fermented liquid) containing the organic acid after the hydrogen fermentation is transferred to the methane fermentation tank 2 by way of a line L3, so as to be subjected to methane fermentation.

The methane fermentation tank 2 contains a methane-fermenting microorganism. A methane-fermenting microorganism group is usually an ecosystem in which a plurality of species of methane-generating bacteria exist. When various methane-generating bacteria such as Methanobacterium, Methanobrevibacter, Methanosarcina, Methanothrix, Methanogenium, and Methanoculles are allowed to live in this ecosystem, methane generation can be performed efficiently. As a consequence, the liquid to be processed (fermented liquid) transferred to the methane fermentation tank 2 after the hydrogen fermentation is decomposed into methane and carbon dioxide. Providing a methane fermentation step after a hydrogen fermentation step as such is quite useful from the viewpoints of reducing the volume of organic wastes, lowering the environmental load due to organic waste water, etc. in addition to the fact that methane can be obtained as an energy gas.

The liquid to be processed (fermented liquid) subjected to the methane fermentation preferably contains a hop or hop component. The fermented liquid containing a hop or hop component is preferable since it can effectively suppress activities of microorganisms which may inhibit the methane fermentation caused by the methane-fermenting microorganism. When a hop or hop component is added to the liquid to be processed at the time of hydrogen fermentation, the hop or hop component is brought into the methane fermentation tank 2 together with the liquid to be processed. However, a hop or hop component may newly be added to the liquid to be processed when the latter is transferred to the methane fermentation tank 2.

The biogas generated by the methane fermentation is a mixed gas of methane and carbon dioxide, and is taken out of the methane fermentation tank 2 by way of a line L4. Though the biogas can be utilized as an energy gas while still in the mixed gas of methane and carbon dioxide, a film separator which passes methane therethrough but not carbon dioxide or an alkali solution absorbing carbon dioxide or the like can yield methane with a high purity. On the other hand, the fermentation liquid residue after the methane fermentation is discharged from the methane fermentation tank 2 by way of a line L5. The fermentation liquid residue is one having sufficiently reduced its volume or detoxified.

The present invention is not restricted to the above-mentioned embodiment. For example, though the above-mentioned embodiment includes a step of determining the maximum tolerable concentration of the substrate consumable by the hydrogen-fermenting microorganism according to the correlation with the rate at which the substrate is consumed by the hydrogen-fermenting microorganism, this step is not always necessary when a hop or hop component is added to the liquid to be processed. Namely, by adding a hop or hop component into the liquid to be processed containing an organic matter and deactivating contaminant microorganisms inhibiting hydrogen generation without affecting the growth or activity of the hydrogen-fermenting microorganism, the present invention can effectively generate a biogas mainly composed of hydrogen.

Though the above-mentioned embodiment relates to hydrogen/methane two-stage fermentation by a continuous operation, the fermenting/cultivating operation of the hydrogen-fermenting microorganism may be not only a continuous operation but also a batch operation, a semibatch operation, and the like. The semibatch operation is an operation in which a specific limiting substrate is supplied to a reactor whereas the aimed product is not taken out until a harvest. This operation is also known as feeding. The batch operation and semibatch operation are favorable in terms of keeping the material concentration within an appropriate range, since the substrate concentration in the fermentation material liquid is easily calculated from the added liquid amount, the substrate concentration in the added liquid, the culture liquid amount in the fermentation tank, and the substrate concentration in the liquid. In the continuous operation, the fermentation material liquid is continuously supplied, while the solution is continuously discharged from within the fermentation tank, whereby the fermentation material liquid is required to be supplied continuously while taking account of the flow-in, flow-out, and material consumption by microorganisms. In general, the purpose of fermentation for collecting an energy gas from a biomass as a material is waste processing of biomasses such as organic resource wastes and organic waste water or waste water processing, whereby the continuous operation is rational in terms of apparatus operating efficiency.

## Examples

In the following, the present invention will be explained more specifically with reference to Examples, which do not restrict the present invention at all.

[Hydrogen Fermentation Inhibiting Action by Lactic Acid Bacteria]

**Example 1**

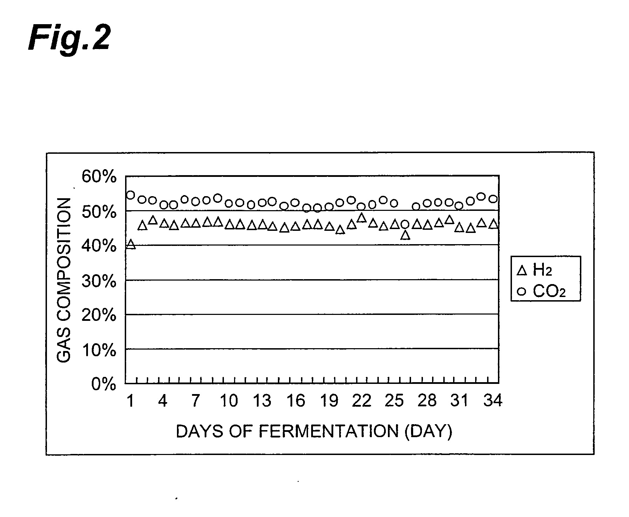

Sludge collected from an anaerobic sludge bed was acclimated in beer brewery waste water (with a pH of 4, COD of about 15000, glucide concentration (calculated as glucose) of 4000 to 5000 mg/L, a lactic acid concentration of about 4000 mg/L, and an acetic acid concentration of about 100 mg/L) at 50°C, and methane-fermenting microorganisms were eliminated therefrom, so as to accumulate an acid-generating fermenting microorganism group capable of performing hydrogen fermentation. Using thus accumulated microorganism group as an inoculum, continuous fermentation fed with beer brewery waste water as a fermentation material liquid was performed for about 1 month. The continuous fermentation was carried out under a condition of pH 6.0 to 6.5 at 50°C. Fig. 2 shows the correlation between the number of days of fermentation and the hydrogen and carbon dioxide concentrations in the fermentation gas. The organic acid generated at the time of hydrogen fermentation was mainly composed of about 1000 mg/L of acetic acid, about 2000 mg/L of butyric acid, and about 200 mg/L of lactic acid. The fermented liquid was collected from a continuous fermentation tank, and was cultivated at 50°C in a culture medium in which the beer brewery waste water was solidified with agar, whereby several species of microorganism colonies were detected as dominant species in the culture liquid. Eight species of microorganisms predominant in the colonies were cultivated in an agar culture medium for anaerobic fermentation, and base sequences of genes in grown colonies were analyzed, whereby five out of the eight species were microorganisms of genus Clostridium. The same eight species of microorganisms were cultivated in an agar culture medium for detecting lactic acid bacteria (modified GAM culture medium available from Nissui Pharmaceutical Co., Ltd.), but no lactic acid bacteria were grown.

**Comparative Example 1**

After 20 g of glucose (made by Wako Pure Chemical Industries), 3 g of yeast extract (made by DIFCO), 5 g of peptone (made by DIFCO), 3 g of malt extract (made by DIFCO), and 5 g of NaHCO$_3$ (made by Wako Pure Chemical Industries) were dissolved in 1 L of tap water, the resulting solution was subjected to steam sterilization at 121°C for 15 minutes, whereby a fermentation material liquid was prepared. The fermentation material liquid was inoculated with a culture liquid which had been obtained by continuously fermenting the beer brewery waste water for 1 month under the condition of pH 6.0 to 6.5 at 50°C as a fermentation material liquid in Example 1, and batch fermentation was repeated for 24 hours each at 50°C. About 60% of hydrogen and about 40% of carbon dioxide were obtained in the first batch fermentation, about 50% of hydrogen and about 50% of carbon dioxide were obtained in the second batch fermentation, and about 35% of hydrogen and about 65% of carbon dioxide were obtained in the third batch fermentation, whereby the hydrogen production rapidly decreased. The amounts of generation of acetic acid, butyric acid, and lactic acid were analyzed before the fermentation and at the respective times when the first, second, and third batch fermentation sessions of the fermentation liquid material ended, whereby lactic acid was found to increase in the third batch fermentation as shown in Table 1. The microorganism group in the third batch cultivation was anaerobically cultivated at 50°C in an agar culture medium comprising glucose, yeast extract, peptone, malt extract, and NaHCO$_3$ (the fermentation liquid material having 15 g of agar added thereto), whereby several species of microorganism colonies were detected as dominant species. Nine species of microorganisms predominant in the colonies were cultivated in an agar culture medium for detecting lactic acid bacteria, and base sequences of genes of grown colonies were analyzed, whereby it was found that, of the nine species, two species were Lactococcus lactis, two species were Enterococcus faecalis, and one species was a species related to Enterococcus avium or the like. This indicated that the increase in the lactic acid bacteria group and the suppression of hydrogen generation occurred in conjunction with each other.

Table 1

|  | Acetic acid (mg/L) | Butyric acid (mg/L) | Lactic acid (mg/L) |
|---|---|---|---|
| Before fermentation | 48 | 0 | 33 |
| End of 1st session | 2600 | 1800 | 380 |
| End of 2nd session | 2800 | 1900 | 190 |
| End of 3rd session | 2100 | 1400 | 3700 |

Comparing Example 1 and Comparative Example 1 with each other showed that predominantly growing microorganism groups varied when properties of materials differed from each other even if the same inoculum was used. These two kinds of material liquids greatly differed from each other in terms of glucide concentration. Namely, it was suggested that predominantly growing microorganism species influenced the glucide concentration of fermented liquids.

[Hydrogen Fermentation Using Beer Brewery Waste Water]

**Example 3**

While changing the dilution ratio of a material liquid prepared by adding an easily assimilatable glucide composed of maltose and starch to beer brewery waste water, hydrogen fermentation by a continuous operation was performed with the same culture liquid as that of Example 1 as an inoculum.

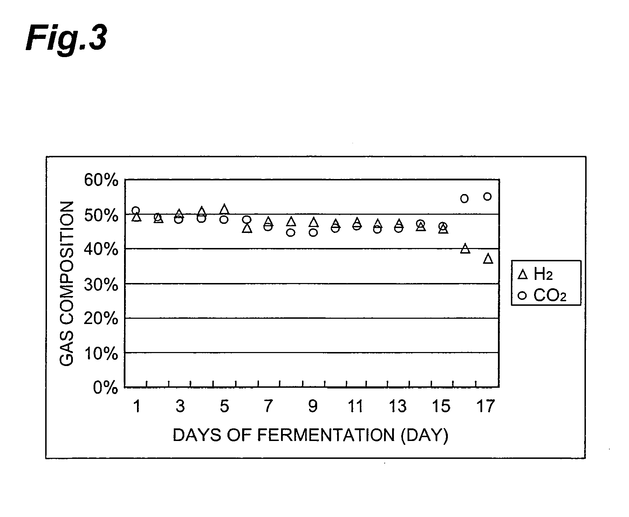

First, when the hydrogen fermentation was carried out with a glucide concentration of about 10000 mg/L in the material liquid and a dilution ratio of 1.0/d in the continuous fermentation (days 1 to 7), the glucide concentration in a hydrogen fermentation tank became stable in the vicinity of 800 mg/L, and the hydrogen fermentation underwent steadily. Thereafter, when the glucide concentration of the material liquid was set to about 22000 mg/L whereas the dilution ratio was 0.4/d (days 8 to 13), the hydrogen fermentation still underwent steadily although the glucide concentration in the hydrogen fermentation tank slightly rose to about 1000 mg/L. When the dilution ratio was set to 1.2/d in the material liquid having about the same glucide concentration (days 14 to 17), the glucide concentration in the hydrogen fermentation tank became about 3800 mg/L, and the amount of hydrogen generation decreased drastically. Namely, when the glucide in the hydrogen fermentation tank was left without being consumed completely, the amount of hydrogen generation decreased, and the lactic acid concentration increased. Fig. 3 shows the correlation between the number of days of fermentation and the hydrogen and carbon dioxide concentrations in the fermentation gas in the above-mentioned hydrogen fermentation. Table 2 shows the glucide concentration of the material liquid, dilution ratio, glucide concentration in the hydrogen fermentation tank, and concentrations of organic acids (acetic acid, butyric acid, and lactic acid) in each period. In Table 2, the glucide concentration and organic acid concentration in the hydrogen fermentation tank in each period are their central values. This result showed that the hydrogen fermentation was kept smoothly within the range where the glucide concentration in the hydrogen fermentation tank did not exceed 4000 mg/L.

Table 2

| Period | Glucide concentration in material liquid (mg/L) | Dilution ratio (1/d) | Glucide concentration in fermentation tank (mg/L) | Organic acid concentration in fermentation tank (mg/L) | | |
| --- | --- | --- | --- | --- | --- | --- |
| | | | | Acetic acid | Butyric acid | Lactic acid |
| Days 1-7 | 10560 | 1.0 | 800 | 1000 | 2500 | 0 |
| Days 8-13 | 22360 | 0.4 | 1000 | 1800 | 6000 | 200 |
| Days 14-15 | 22360 | 1.2 | 3800 | 1500 | 5000 | 4000 |
| Days 16-17 | 24030 | 1.2 | 3800 | 1100 | 3300 | 6000 |

[Hydrogen Fermentation Using Bread Bakery Waste]

**Example 4**

Using liquids in which bread bakery wastes were suspended in water at various concentrations as a material, continuous hydrogen fermentation was performed under the condition of pH 6.0 to 6.5 at 50°C with the same culture liquid as that of Comparative Example 1 as an inoculum.

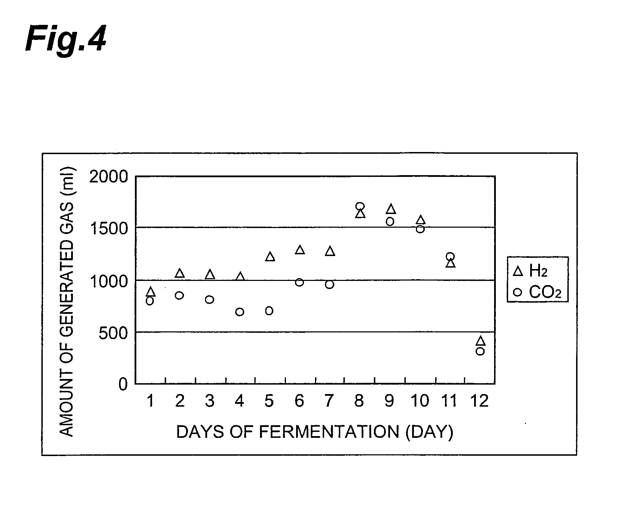

First, when the hydrogen fermentation was carried out with a glucide concentration of about 11000 mg/L in the material liquid and a dilution ratio of 0.7/d in the continuous fermentation (days 1 to 6), the glucide concentration in the fermentation tank was 3000 to 4000 mg/L, whereby the hydrogen fermentation was performed steadily. Next, when a material liquid with a higher bread bakery waste concentration (glucide concentration of about 35000 mg/L in the material liquid) was supplied (days 7 to 12), the hydrogen generation occurred vigorously until 8 days after the higher concentration material liquid supply; on day 9 and thereafter, the amount of hydrogen generation decreased, and the lactic acid concentration rose. In the fermentation period where the hydrogen fermentation was performed smoothly without decreasing the amount of hydrogen generation (days 1 to 6), the glucide concentration in the fermented liquid was 3000 to 4000 mg/L. Fig. 4 shows the correlation between the number of days of fermentation and the hydrogen and carbon dioxide concentrations in the fermentation gas in the above-mentioned hydrogen fermentation. Table 3 shows the glucide concentration of the material liquid, dilution ratio, glucide concentration in the hydrogen fermentation tank, and concentrations of organic acids (acetic acid, butyric acid, and lactic acid) in each period. This elucidated that the hydrogen fermentation could be maintained smoothly when the material concentration in the supplied material liquid was held appropriately in the hydrogen fermentation tank.

Table 3

| Days of fermentation | Glucide concentration in material liquid (mg/L) | Dilution ratio (1/d) | Glucide concentration in fermentation tank (mg/L) | Organic acid concentration in fermentation tank (mg/L) | | |
|---|---|---|---|---|---|---|
| | | | | Acetic acid | Butyric acid | Lactic acid |
| 1 | 11340 | 0.7 | 3050 | 2439 | 2904 | 64 |
| 2 | 11340 | 0.7 | 3470 | 1950 | 2428 | 0 |
| 3 | 11340 | 0.7 | 3390 | 1913 | 2043 | 0 |
| 4 | 11340 | 0.7 | 3110 | 1642 | 1704 | 0 |
| 5 | 11340 | 0.7 | 3590 | 1658 | 1610 | 0 |
| 6 | 11340 | 0.7 | 4060 | 1674 | 1516 | 44 |
| 7 | 34890 | 0.35 | 3960 | 1237 | 1134 | 852 |
| 8 | 34890 | 0.35 | 4470 | 2792 | 1960 | 4106 |
| 9 | 34890 | 0.35 | 4550 | 3258 | 3861 | 4500 |
| 10 | 34890 | 0.35 | 5170 | 3229 | 6997 | 4991 |
| 11 | 34890 | 0.35 | 6850 | 2696 | 7826 | 5488 |
| 12 | 34890 | 0.35 | 6640 | 2382 | 9136 | 5824 |

Thus, hydrogen fermentation can be maintained smoothly when a substrate concentration, a glucide concentration in particular, in a fermentation tank is used as an index, and a material liquid is supplied such that this index is adjusted so as to fall within a favorable range. Specifically, when the glucide concentration in the fermented liquid in the fermentation tank is kept at 4000 mg/L or lower in the case of hydrogen-fermenting microorganisms based on beer brewery waste water and bread bakery wastes, lactic acid bacteria groups remarkably inhibiting the hydrogen-fermenting microorganisms can be restrained from predominantly increasing, whereby the hydrogen fermentation can be maintained smoothly.

**Example 5**

While controlling the material liquid supply rate in conformity to changes in the glucide concentration in the fermentation material liquid and keeping the glucide concentration at 3000 mg/L in the hydrogen fermentation tank, hydrogen fermentation was performed under a condition of pH 6.0 to 6.5 at 50°C. Specifically, continuous fermentation was initially performed for about 1 month with the same culture liquid as that of Comparative Example 1 as an inoculum in a material liquid (whose total glucide concentration was 10710 mg/L to 18390 mg/L) prepared by adding maltose and starch to beer brewery waste water in order to enhance the microorganism concentration in the fermentation tank. Thereafter, using material liquids prepared by adding maltose and starch to the beer brewery waste water or material liquids in which bread bakery wastes were suspended in water at various concentrations, continuous hydrogen fermentation in which the liquid supply rate was controlled so as to keep a constant glucide concentration in the fermentation tank was performed. In the fermentation of about 1 month carried out before performing the continuous fermentation with controlled liquid supply rate, a value of about 7500 mg/L/day was obtained as the glucide consumption capacity ($-dS/dt)_C$ of this fermentation system. This value was used for determining a control value for material liquid supply rate in expression (3b). Since the glucide concentration in the fermentation tank was required to be about 4000 mg/L or less in order to keep hydrogen fermentation as evidenced by Examples 3 and 4, the control glucide concentration S in the fermentation tank was set to 3000 mg/L. Using these values and the supplied material liquid glucide concentration, a control index value for the rate at which the material liquid was supplied to the fermentation tank was calculated by expression (3b). Table 4 shows control index values for material liquid glucide concentrations. In the hydrogen fermentation with the controlled material liquid supply rate, continuous fermentation was performed 4 days for a material liquid, and then was continuously switched to material liquids with different concentrations. Table 4 shows the values at 3 and 4 days after switching the material liquids. The actual dilution ratios in Table 4 are values calculated from actual material liquid supply amounts. The glucide concentration in the fermentation tank was near an initial target of 3000 mg/L, and the amount of hydrogen generation was substantially proportional to the amount of glucide consumption. This showed that the hydrogen fermentation was maintained smoothly.

Table 4

| Glucide concentration in material liquid (mg/L) | Control index dilution ratio (1/d) | Days of fermentation | Actual dilution ratio (1/d) | Glucide concentration in fermentation tank (mg/L) | Amount of hydrogen generated (mL) | Amount of hydrogen generated per unit amount of glucide consumed (mL/mg) |
|---|---|---|---|---|---|---|
| 11160 | 0.92 | 3 | 0.90 | 2689 | 1410 | 0.20 |
| 11160 | 0.92 | 4 | 0.90 | 3038 | 1366 | 0.18 |
| 8820 | 1.29 | 3 | 1.21 | 3120 | 1269 | 0.19 |
| 8820 | 1.29 | 4 | 1.21 | 3314 | 1328 | 0.20 |
| 10560 | 0.99 | 3 | 0.95 | 2823 | 1259 | 0.17 |
| 10560 | 0.99 | 4 | 0.95 | 2771 | 1366 | 0.19 |
| 22360 | 0.39 | 3 | 0.37 | 2984 | 1479 | 0.21 |
| 22360 | 0.39 | 4 | 0.37 | 3136 | 1383 | 0.19 |
| 38280 | 0.21 | 3 | 0.20 | 3359 | 1410 | 0.21 |
| 38280 | 0.21 | 4 | 0.20 | 3549 | 1527 | 0.21 |

(Effectiveness of Hop and Hop Component)

**Example 6**

After 20 g of glucose (made by Wako Pure Chemical Industries), 3 g of yeast extract (made by DIFCO), 5 g of peptone (made by DIFCO), 3 g of malt extract (made by DIFCO), and 1 g of hop pellets (Hop Pellets Type 90 manufactured by Botanix) were dissolved in 1 L of tap water, thus obtained solution was subjected to steam sterilization at 121 °C for 15 minutes, whereby a fermentation material liquid was prepared. Subsequently, the fermentation material liquid was inoculated with the same culture liquid as that of Comparative Example 1 as an inoculum, batch fermentation at 50°C was repeated eight times for 24 hours each. As a result, the fermentation gas composition was composed of about 53% of hydrogen and about 40% of carbon dioxide in all the batch fermentation sessions, whereby hydrogen production was maintained. When compositions of organic acids generated at that time were analyzed, no great changes were seen in eight batch fermentation sessions (Table 5). Though the glucide concentration in the fermented liquid was high, contaminant microorganism groups did not increase in the hydrogen fermentation, whereby the hydrogen fermentation was not obstructed. This elucidated that, unlike Comparative Example 1, the addition of the hop component inhibited activities of microorganism groups having adverse affects of suppressing the growth or hydrogen generation of hydrogen-fermenting microorganisms, but did not obstruct activities of the hydrogen-fermenting microorganisms.

Table 5

| Number of fermentations | Glucide concentration after fermentation (mg/L) | Organic acid concentration after fermentation (mg/L) | | |
|---|---|---|---|---|
| | | Acetic acid | Butyric acid | Lactic acid |
| 2 | 7528 | 1843 | 2363 | 80 |

(continued)

| Number of fermentations | Glucide concentration after fermentation (mg/L) | Organic acid concentration after fermentation (mg/L) | | |
|---|---|---|---|---|
| | | Acetic acid | Butyric acid | Lactic acid |
| 4 | 7267 | 2369 | 2532 | 0 |
| 6 | unanalyzed | 2040 | 2538 | 116 |
| 7 | 9334 | 2042 | 2648 | 0 |
| 8 | unanalyzed | 2175 | 2708 | 75 |

**Example 7**

The fermented liquid of Example 6 was collected, and its bitterness (defined by European Brewery Convention, Analytica-EBC 4th ed., p. E137, 1987) was measured. The bitterness was about 13. This elucidated that the hop component inhibited activities of microorganism groups suppressing the growth or hydrogen generation of hydrogen-fermenting microorganisms at a bitterness near 13, but did not obstruct the activities of the hydrogen-fermenting microorganisms.

**Example 8**

A hop component was added to the culture system of Example 4 having drastically reduced the amount of hydrogen generation, so as to restore its hydrogen generation.

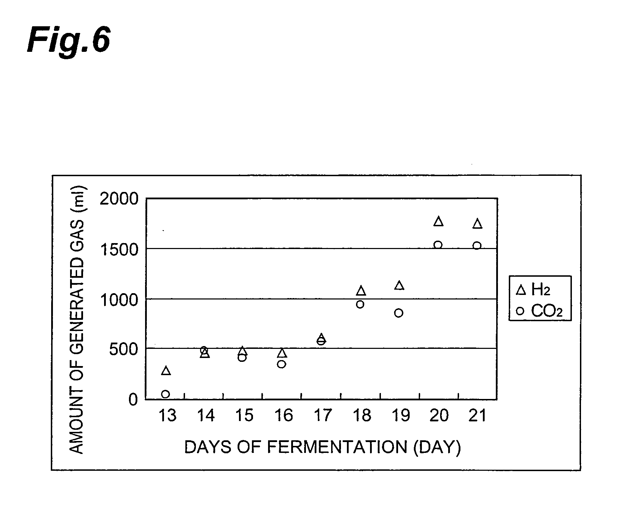

Specifically, a material liquid having reduced the glucide concentration of the supply liquid was initially supplied to the culture system of Example 4 from day 13, and an operation was performed for 3 days (days 13 to 15). However, this operation did not restore the hydrogen fermentation, whereby the amount of hydrogen gas generation did not recover. Therefore, on day 16, hop pellets (Hop Pellets Type 90 manufactured by Botanix) were added to the fermentation tank and the supply liquid by 1 g per 1 L of the fermented liquid. The hydrogen production exhibited a tendency to recover on day 17 and thereafter, and was restored to the level at the time of starting the higher concentration material liquid supply on day 20 (Fig. 6). On day 19 and thereafter, the generated organic acid composition was restored to the level at the time of starting the higher concentration material liquid supply (Table 6). This elucidated that a hop as pellets at a concentration of 1 g per 1 L of fermented liquid inhibited activities of unfavorable microorganism groups suppressing the growth or hydrogen generation of hydrogen-fermenting microorganisms, but did not obstruct activities of the hydrogen-fermenting microorganisms.

Table 6

| Period | Glucide concentration in material liquid (mg/L) | Dilution ratio (1/d) | Glucide concentration in fermentation tank (mg/L) | Organic acid concentration in fermentation tank (mg/L) | | |
|---|---|---|---|---|---|---|
| | | | | Acetic acid | Butyric acid | Lactic acid |
| 13 | 17010 | 0.55 | unanalyzed | 1089 | 4028 | 3393 |
| 14 | 17010 | 0.55 | 4468 | 1310 | 3330 | 3055 |
| 15 | 17010 | 0.55 | unanalyzed | 1490 | 3768 | 3459 |
| 16 | 17010 | 0.55 | 3959 | 1614 | 3794 | 2706 |
| 17 | 17010 | 0.55 | unanalyzed | 1445 | 3393 | 1975 |
| 18 | 17010 | 0.55 | 8926 | 1446 | 3514 | 936 |
| 19 | 17010 | 0.55 | unanalyzed | 1522 | 3307 | 175 |
| 20 | 17010 | 0.55 | unanalyzed | 2154 | 3345 | 92 |
| 21 | 17010 | 0.55 | unanalyzed | 2679 | 3122 | 72 |

**Example 9**

For various hop components, effects of smoothly maintaining hydrogen fermentation were investigated.

After 35 g of glucose (special grade reagent made by Wako Pure Chemical Industries), 3 g of yeast extract (made by DIFCO), 5 g of peptone (made by DIFCO), 3 g of malt extract (made by DIFCO), and at least one species of hop components shown in Table 7 were dissolved in 1 L of tap water, the resulting solution was subjected to steam sterilization at 121°C for 15 minutes, whereby fermentation material liquids A to F were prepared. On the other hand, fermentation material liquid G was made in the same manner except that no hop component was added.

Table 7

| Fermentation material liquid | Hop component | Amount added | Bitterness of fermented material liquid |
|---|---|---|---|
| A | Hop pellets (Pellets Type 90 made by Botanix) | 1g | 13 |
| B | Hop extract (EX made by Kalsec) | 3.5g | 12 |
| C | Isomerized hop pellets (Isomerised Hop Pellets made by Botanix) | 0.5g | 11 |
| D | Tetrahop (Tetralone made by Kalsec) | 180μL | 11 |
| E | Hop α-acid (Isohop made by Botanix) | 50μL | 12 |
| F | Hop β-acid (Beta Stab 10A made by Beta Tec) | 10μL | - |
| G | No addition | - | 0 |

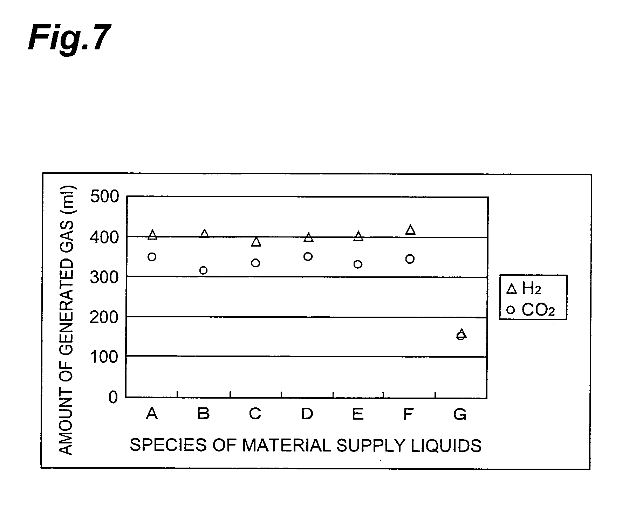

Next, each of the fermentation material liquids A to G was inoculated with the same culture liquid as that of Example 1 as an inoculum, and batch fermentation was repeated four times for 24 hours each. Though the hydrogen production decreased while lactic acid increased in the sample to which no hop component was added, about 400 ml of hydrogen and about 350 ml of carbon dioxide were attained in all the batch fermentation sessions in the samples to which hop components were added without lowering the hydrogen production (Fig. 7). Lactic acid did not increase in any of the samples to which hop components were added (Table 8). This elucidated that, except for the beta acid exhibiting no bitterness, an amount of addition of hop components by a bitterness of 10 or greater inhibited activities of microorganism groups having adverse affects of suppressing the growth or hydrogen generation of hydrogen-fermenting microorganisms, but did not obstruct activities of the hydrogen-fermenting microorganisms. At an amount of addition of 10 μL per 1 L of fermented liquid, the beta acid was found to inhibit activities of microorganism groups having adverse affects of suppressing the growth or hydrogen generation of hydrogen-fermenting microorganisms, but did not obstruct activities of the hydrogen-fermenting microorganisms

Table 8

| Fermentation material liquid | Glucide concentration after fermentation (mg/L) | Organic acid concentration after fermentation (mg/L) | | |
|---|---|---|---|---|
| | | Acetic acid | Butyric acid | Lactic acid |
| A | 16423 | 2390 | 3353 | 273 |
| B | 15916 | 2321 | 4613 | 149 |
| C | 15746 | 2376 | 4854 | 170 |
| D | 12491 | 2317 | 5172 | 109 |
| E | 14211 | 2005 | 4793 | 91 |

(continued)

| Fermentation material liquid | Glucide concentration after fermentation (mg/L) | Organic acid concentration after fermentation (mg/L) | | |
|---|---|---|---|---|
| | | Acetic acid | Butyric acid | Lactic acid |
| F | 15546 | 2069 | 4409 | 61 |
| G | 17359 | 3515 | 4590 | 2191 |

**Example 10**

It was also found that subjecting a fermented liquid after hydrogen fermentation of a biomass material having a hop or hop component added thereto or contained therein to methane fermentation caused by a methane-fermenting microorganism smoothly maintained the methane fermentation. This will be shown.

The hydrogen fermentation effluent in which the hydrogen fermentation material containing hop pellets was supplied to the fermentation system of Example 1 in which the hydrogen fermentation was contaminated with microorganisms inhibiting the hydrogen fermentation and thus lowered the hydrogen production, so as to restore the hydrogen fermentation, was subjected to methane fermentation by methane-fermenting microorganisms, and it was tested whether the methane fermentation was maintained smoothly or not.

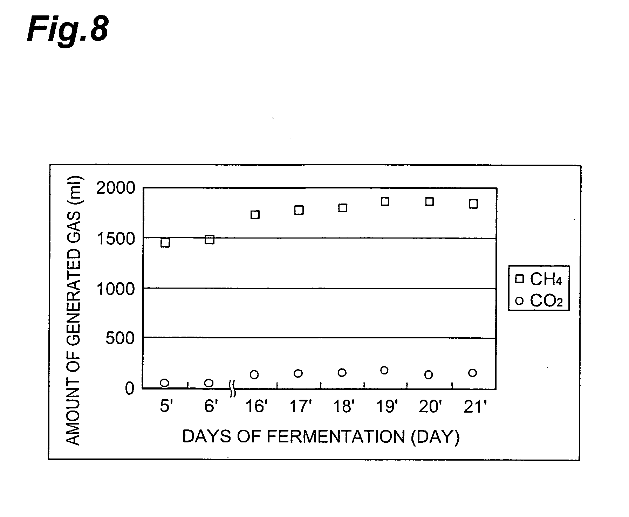

First, the fermentation effluent in which hydrogen fermentation progressed normally without no hop component added thereto in Example 4 was subjected to methane fermentation under a condition of pH 7.0 to 7.5 at 37°C. Namely, using the effluents on days 5 and 6 of hydrogen fermentation in Example 4 as a hydrogen fermentation effluent (methane fermentation material liquid), the methane fermentation was performed. When supplying the material liquid to the methane fermentation, the dilution ratio was 0.43/d. Fig. 8 shows thus obtained results (days 5' and 6' in Fig. 8).

Thereafter, the hydrogen fermentation effluent after performing the hydrogen fermentation using the fermentation material liquid A of Example 8 (having hop pellets added thereto) was subjected to methane fermentation. Namely, using the effluents on days 16 to 21 of hydrogen fermentation in Example 8 as a hydrogen fermentation effluent (i.e., methane fermentation material liquid), the methane fermentation was performed. When supplying the material liquid to the methane fermentation, the dilution ratio was 0.40/d. Fig. 8 shows thus obtained results (days 16' and 21' in Fig. 8).

As shown in Fig. 8, the methane fermentation caused by the methane-fermenting microorganism exhibited no abnormality in the amount of methane generation even when using the effluents obtained after performing the hydrogen fermentation by the hydrogen fermentation materials containing hop pellets. This has elucidated that methane fermentation caused by methane-fermenting microorganisms is smoothly maintained also when fermented liquid obtained after performing hydrogen fermentation with a biomass material having a hop or hop component added thereto or contained therein is subjected to the methane fermentation.

**Claims**

1.    A production method of a biogas, the method comprising:

a first step of determining, according to a correlation between a concentration of a predetermined substrate in a liquid to be processed containing an organic matter and a rate of consumption of the substrate by a hydrogen-fermenting microorganism, a maximum tolerable concentration of the substrate consumable by the hydrogen-fermenting microorganism; and
a second step of generating a biogas mainly composed of hydrogen by causing the hydrogen-fermenting microorganism to hydrogen-ferment the liquid to be processed while keeping the substrate in the liquid to be processed at a concentration not higher than the maximum tolerable concentration.

2.    A production method of a biogas according to claim 1, wherein the substrate is a glucide.

3.    A production method of a biogas according to claim 1 or 2, further comprising a third step of generating a fermentation gas mainly composed of methane by causing a methane-fermenting microorganism to methane-ferment the fermented liquid after the hydrogen fermentation in the second step.

4.    A production method of a biogas, the method comprising the step of generating a biogas mainly composed of hydrogen by performing hydrogen fermentation while adding a hop or hop component to a liquid to be processed containing an organic matter so as to inactivate a contaminant microorganism inhibiting hydrogen generation without affecting a growth or activity of a hydrogen-fermenting microorganism.

Fig.1

# Fig.2

# *Fig.3*

# *Fig.4*

# *Fig.5*

*Fig.6*

# Fig.7

*Fig.8*

## INTERNATIONAL SEARCH REPORT

| | International application No. |
|---|---|
| | PCT/JP2005/002226 |

**A. CLASSIFICATION OF SUBJECT MATTER**
Int.Cl$^7$ C02F11/04, 3/00, 3/28, C12P3/00, 5/02, B09B3/00, C10L3/00

According to International Patent Classification (IPC) or to both national classification and IPC

**B. FIELDS SEARCHED**

Minimum documentation searched (classification system followed by classification symbols)
Int.Cl$^7$ C02F11/04, 3/00, 3/28, C12P3/00, 5/02, B09B3/00, C10L3/00

Documentation searched other than minimum documentation to the extent that such documents are included in the fields searched
Jitsuyo Shinan Koho        1926-1996   Toroku Jitsuyo Shinan Koho   1994-2005
Kokai Jitsuyo Shinan Koho   1971-2005   Jitsuyo Shinan Toroku Koho    1996-2005

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 05-096294 A  (Ebara Research Co., Ltd.), 20 April, 1993 (20.04.93), Full text; Fig. 1 (Family: none) | 1-4 |
| A | JP 07-136694 A  (Ebara Research Co., Ltd.), 30 May, 1995 (30.05.95), Full text; Figs. 1 to 4 (Family: none) | 1-4 |
| A | JP 2003-116589 A  (Tadayuki IMANAKA), 22 April, 2003 (22.04.03), Full text; Fig. 1 (Family: none) | 1-4 |

| [X] | Further documents are listed in the continuation of Box C. | [ ] | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 March, 2005 (24.03.05) | 12 April, 2005 (12.04.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| International application No. |
|---|
| PCT/JP2005/002226 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2003-135089 A  (Takuma Co., Ltd.),<br>13 May, 2003 (13.05.03),<br>Full text; Fig. 1<br>(Family: none) | 1-4 |
| P,Y | JP 2004-194625 A  (Densei Inc.),<br>15 July, 2004 (15.07.04),<br>Full text; Figs. 1 to 6<br>(Family: none) | 1-3 |
| P,Y | JP 2005-013045 A  (Takuma Co., Ltd.),<br>20 January, 2005 (20.01.05),<br>Full text; Figs. 1 to 10<br>(Family: none) | 1-3 |
| E,Y | JP 2005-066420 A  (Japan Science and<br>Technology Agency),<br>17 March, 2005 (17.03.05),<br>Full text; Figs. 1 to 8<br>(Family: none) | 1-3 |

Form PCT/ISA/210 (continuation of second sheet) (January 2004)

## INTERNATIONAL SEARCH REPORT

| International application No. |
| --- |
| PCT/JP2005/002226 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
   The matter common to the inventions of claims 1-3 and claim 4 is to carry out hydrogen fermentation of a subject solution containing organic matter with the use of a hydrogen fermentation microbe to thereby produce a biogas composed mainly of hydrogen.
   However, this common matter is not a special technical feature as disclosed in, for example, the reference mentioned in the section of prior art by the applicant, JP 2001-149983 A (Naomichi NISHIO) 05 June, 2001 (05.06.01), claim 1.
   Consequently, there is no real commonality between the inventions of claims 1-3 and claim 4.                              (continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐    The additional search fees were accompanied by the applicant's protest.

                             ☐    No protest accompanied the payment of additional search fees.

Form PCT/ISA/210 (continuation of first sheet (2)) (January 2004)

**INTERNATIONAL SEARCH REPORT**

| International application No. |
| --- |
| PCT/JP2005/002226 |

Continuation of Box No.III of continuation of first sheet(2)

Therefore, the inventions of claims 1-3 and claim 4 do not satisfy the requirement of unity of invention.

Form PCT/ISA/210 (extra sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 61008200 A
- JP 2001149983 A
- JP 2003135089 A

**Non-patent literature cited in the description**

- **NOIKE et al.** Inhibition of hydrogen fermentation of organic wastes by lactic acid bacteria. *International Journal of Hydrogen Energy,* 2002, vol. 27, 1367-1371
- **SIMPSON, W.J. et al.** Factors affecting antibacterial activity of hops and their derivatives. *J. Appl. Bacteriol.,* 1992, vol. 72, 327-334
- **PLOLLACH G et al.** Einsatz von Hophenprodukten als Bacteriostaticum in der Zuckerindustrie. *Zuckerindustrie,* 1996, vol. 121, 919-926
- **HEIN, W. et al.** Neue Erkenntnisse beim Einsatz von Hopfenprodukten in der Zuckerindustrie. *Zuckerindustrie,* 1997, vol. 122, 940-949
- **PLOLLACH, G et al.** Neue Erkenntnisse zur Losungmikrobieller Probleme in Zuckerfabriken. *Zuckerindustrie,* 1999, vol. 124, 622-637
- **SIMPSON, W.J. et al.** Cambridge Prize Lecture, Studies on the Sensitivity of Lactic Acid Bacteria to Hop Bitter Acids. *J. Inst. Brew.,* 1993, vol. 99, 405-411
- **SAMI, M.** Lactic Acid Bacteria Deteriorating Beer. *Journal of the Brewing Society of Japan,* 1999, vol. 94, 2-9